# EUROPEAN PATENT APPLICATION

(11) **EP 4 623 826 A1**
(43) Date of publication of application: **01.10.2025**
(21) Application number: 23903779.9
(22) Date of filing: 14.11.2023
(51) Int. Cl.: A61B 5/349, A61B 5/00, G16H 50/20

(54) **METHOD, PROGRAM, AND DEVICE FOR QUANTIFYING QUALITY OF BIOSIGNALS**

(30) Priority: 14.12.2022 KR 20220174508
(71) Applicant: Medical AI Co., Ltd., Seoul 06180 (KR)
(72) Inventor: KWON, Joonmyoung, Seoul 06180 (KR); SON, Jeongmin, Seoul 06180 (KR); PARK, Taejun, Seoul 06180 (KR)
(74) Representative: Patentanwaltskanzlei Matschnig & Forsthuber OG
(86) International application number: PCT/KR2023/018263
(87) International publication number: WO 2024/128576

(57) **Abstract**

Disclosed herein are a method, program and device for quantifying the quality of biosignals according to one embodiment of the present disclosure. The method may include: obtaining at least one of a first electrocardiogram dataset labeled based on morphological features of electrocardiogram signals and a second electrocardiogram dataset labeled based on the analysis of a domain expert on the reading of electrocardiogram signals; and generating a machine learning model for quantifying the quality of electrocardiogram signals based on at least one of the first and second electrocardiogram datasets.

## Description

### Technical Field

The present disclosure relates to deep learning technology in the medical field, and more specifically, to a method and device that can quantify the readability of biosignals with the readability reflected in quality analysis.

### Background Art

In order to utilize signal-based data in application fields, it is important to evaluate the quality of collected signals. Accordingly, measures for evaluating the quality of signals are being studied in various application fields such as acoustics, communications, optics, medicine, etc. The measures for evaluating the quality of signals being studied in various application fields as described above are collectively called signal quality indices (SQI).

Most of the previously studied signal quality indices reflect the quantity of noise or artifacts having occurred in a signal itself in the quality of the signal. However, in the medical field, there are many cases where it is not difficult to use a signal for clinical determination, such as the diagnosis or prediction of disease, even when the signal has a lot of noise or artifacts. In other words, even in the case where the quality of a signal is evaluated as poor due to noise or artifacts according to the previously studied signal quality indices, when the signal contains sufficient information necessary for clinical reading, it needs to be evaluated as the data that can be used in the medical field. Therefore, in the medical field, when the quality of a signal is evaluated, it is necessary to reflect whether the signal is clinically readable in the evaluation.

For example, assuming that the quality of an electrocardiogram signal is evaluated in order to use an electrocardiogram to diagnose heart disease A, even an electrocardiogram signal that is determined to be poor in quality based on the conventional signal quality index may contain all the information necessary to diagnose heart disease A. Accordingly, even when the quality of a signal is poor based on a reference signal quality index, it is inappropriate to evaluate the signal as unusable because it lacks information necessary to diagnose heart disease A. In other words, the quality of a signal should be evaluated according to the purpose of utilization of the signal, so that it is necessary to establish a signal quality index that is optimized for the purpose of utilization in the medical field.

### Disclosure

### Technical Problem

The present disclosure is directed to the provision of a method and device for quantifying signal quality that can reflect the clinical readability of biosignals therein. In addition, the present disclosure is directed to the provision of a method and device that can generate a reliable machine learning model for the above-described quantification of quality.

However, the objects to be achieved in the present disclosure are not limited to the object mentioned above, and other objects not mentioned may be clearly understood based on the following description.

### Technical Solution

According to one embodiment of the present disclosure for achieving the above-described object, there is disclosed a method of quantifying the quality of biosignals that is performed by a computing device. The method may include: obtaining at least one of a first electrocardiogram dataset labeled based on morphological features of electrocardiogram signals and a second electrocardiogram dataset labeled based on the analysis of a domain expert on the reading of electrocardiogram signals; and generating a machine learning model for quantifying the quality of electrocardiogram signals based on at least one of the first and second electrocardiogram datasets.

Alternatively, the first electrocardiogram dataset may be labeled as a first class indicating that an electrocardiogram signal is a readable signal or a second class indicating that an electrocardiogram signal is an unreadable signal based, on the noise identified based on morphological features of the electrocardiogram signal.

Alternatively, the second class may correspond to at least one of: a case where the noise that prevents at least one of the start and end points of the waveform of the electrocardiogram signal from being identified is present at a predetermined ratio or more; and a case where the noise that prevents the R peak of the electrocardiogram signal from being identified is present at a predetermined ratio or more.

Alternatively, the second electrocardiogram dataset may be labeled based on the modes of analyses of the domain expert on whether the noise present in the electrocardiogram signals affects the diagnosis of disease.

Alternatively, any one of the first and second electrocardiogram datasets may be divided into and used as a training dataset, a validation dataset, and a test dataset to generate the machine learning model. Furthermore, the other one of the first and second electrocardiogram datasets may be used as a test dataset to generate the machine learning model.

Alternatively, the machine learning model may include: a first model based on a neural network for estimating the readability of electrocardiogram signals based on an electrocardiogram dataset; and a second model based on regression analysis for estimating the readability of electrocardiogram signals based on an electrocardiogram dataset.

Alternatively, generating the machine learning model for quantifying the quality of electrocardiogram signals based on at least one of the first and second electrocardiogram datasets may include: training candidate models of the first model based on a first training dataset included in the first electrocardiogram dataset; validating the performance of the trained candidate models based on a first validation dataset included in the first electrocardiogram dataset; evaluating the performance of at least one candidate model, selected through the validation, based on a first test dataset which is included in the first electrocardiogram dataset and a second test dataset which is the second electrocardiogram dataset; and generating the first model based on the specifications of the candidate model identified through the evaluation.

Alternatively, generating the machine learning model for quantifying the quality of electrocardiogram signals based on at least one of the first and second electrocardiogram datasets may include: extracting information about signal quality indices (SQI) used to evaluate the noise of electrocardiogram signals from the first electrocardiogram dataset, and generating an index dataset; training candidate models of the second model based on a third training dataset included in the generated index dataset; validating the performance of the trained candidate models based on a third validation dataset included in the generated index dataset; evaluating the performance of at least one candidate model, selected through the validation, based on a third test dataset included in the generated index dataset; and generating the second model based on the specifications of the candidate model identified through the evaluation.

According to one embodiment of the present disclosure for achieving the above-described object, there is disclosed a method of quantifying quality of biosignals that is performed by a computing device. The method may include: obtaining reading target data; and computing a score indicating the readability of the reading target data by inputting the obtained reading target data to a machine learning model. In this case, the machine learning model may be generated based on at least one of a first electrocardiogram dataset labeled based on morphological features of electrocardiogram signals and a second electrocardiogram dataset labeled based on the analysis of a domain expert on the reading of electrocardiogram signals.

Alternatively, the method may further include: estimating whether a signal included in the obtained reading target data is missing; and determining whether the obtained reading target data is readable data by combining the computed score and the estimated presence or absence of a missing signal.

Alternatively, the presence or absence of a missing signal may be estimated based on whether a signal value included in the obtained reading target data is blank at a predetermined ratio or more or whether the waveform of a signal included in the obtained reading target data is flat.

Alternatively, determining whether the obtained reading target data is readable data by combining the computed score and the estimated presence or absence of a missing signal may include: determining whether the noise is present in a signal included in the obtained reading target data by comparing the computed score with a threshold value; and determining whether the obtained reading target data is readable data based on at least one of the presence or absence of noise in the signal determined based on the computed score and the presence or absence of a missing signal estimated above.

Alternatively, determining whether noise is present in the signal included in the obtained reading target data by comparing the computed score with the threshold value may include: determining that noise is present in a signal of a specific lead when a computed score based on the specific lead is equal to or higher than the threshold value.

Alternatively, determining whether the obtained reading target data is readable data based on at least one of the presence or absence of noise in the signal determined based on the computed score and the presence or absence of a missing signal estimated above may include: determining that a signal of a specific lead determined to have noise based on the computed score or estimated as a mission signal is unreadable data.

According to one embodiment of the present disclosure for achieving the above-described object, there is disclosed a computer program stored in a computer-readable storage medium. The computer program causes operations for quantifying the quality of biosignals to be performed when executed by at least one processor. The operations may include operations of: obtaining at least one of a first electrocardiogram dataset labeled based on morphological features of electrocardiogram signals and a second electrocardiogram dataset labeled based on the analysis of a domain expert on the reading of electrocardiogram signals; and generating a machine learning model for quantifying the quality of electrocardiogram signals based on at least one of the first and second electrocardiogram datasets.

According to one embodiment of the present disclosure for achieving the above-described object, there is disclosed a computing device for quantifying the quality of biosignals. The computing device may include: a processor including at least one core; memory including program codes executable by the processor; and a network unit for obtaining at least one of a first electrocardiogram dataset labeled based on morphological features of electrocardiogram signals and a second electrocardiogram dataset labeled based on analysis of a domain expert on reading of electrocardiogram signals. In this case, wherein the processor may generate a machine learning model for quantifying the quality of electrocardiogram signals based on at least one of the first and second electrocardiogram datasets.

### Advantageous Effects

The present disclosure provides the method and device for the quantification of quality that can reflect the readability of biosignals therein. In addition, the present disclosure provides the method and device that can generate a reliable machine learning model for the above-described quantification of quality.

### Description of Drawings

FIG. 1 is a block diagram of a computing device according to one embodiment of the present disclosure;
FIG. 2 is a block diagram showing a process of generating a machine learning model according to one embodiment of the present disclosure;
FIG. 3 is a block diagram showing a process of generating a machine learning model according to an alternative embodiment of the present disclosure;
FIG. 4 is a block diagram showing a process in which a computing device quantifies the quality of biosignals according to one embodiment of the present disclosure;
FIG. 5 is a flowchart showing a method of generating a machine learning model for quantifying the quality of biosignals according to one embodiment of the present disclosure;
FIG. 6 is a flowchart showing a method of quantifying the quality of biosignals according to one embodiment of the present disclosure; and
FIG. 7 is a flowchart summarizing a process of quantifying the quality of biosignals according to one embodiment of the present disclosure.

### Mode for Invention

Embodiments of the present disclosure will be described in detail below with reference to the accompanying drawings so that those having ordinary skill in the art of the present disclosure (hereinafter, those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a machine learning "model" may refer to an overall system that performs operations based on a machine learning algorithm. In this case, the machine learning algorithm may include a classification algorithm such as naive Bayes, a decision tree, etc., a regression analysis algorithm such as linear regression, logistic regression, etc., and a deep learning algorithm such as a convolutional neural network, etc. The types of machine learning algorithms of the present disclosure are not limited to the examples described above, and may be configured in various manners within a range understandable to those skilled in the art based on the content of the present disclosure.

The term "image" used herein may refer to multidimensional data composed of discrete image elements. In other words, "image" may be understood as a term that refers to a digital representation of an object that can be viewed by a human eye. For example, "image" may refer to multidimensional data composed of elements corresponding to pixels in a two-dimensional image. "Image" may refer to multidimensional data composed of elements corresponding to voxels in a three-dimensional image.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

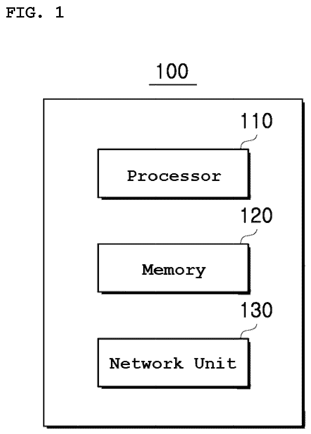

FIG. 1 is a block diagram of a computing device according to one embodiment of the present disclosure.

A computing device 100 according to one embodiment of the present disclosure may be a hardware device or a part of a hardware device that performs the comprehensive processing and computation of data, or may be a software-based computing environment connected over a communication network. For example, the computing device 100 may be a server that is a main agent for performing an intensive data processing function and sharing resources, or may be a client that shares resources through interaction with a server. Alternatively, the computing device 100 may be a cloud system in which multiple servers and clients comprehensively process data while interacting with each other. Since the above description is only one example related to the type of computing device 100, the type of computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 is only an example, and the computing device 100 may further include other components for implementing a computing environment. Furthermore, only some of the disclosed components may be included in the computing device 100.

The processor 110 according to one embodiment of the present disclosure may be understood as a constituent unit including hardware and/or software for performing computing operations. For example, the processor 110 may read a computer program and perform data processing for machine learning. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), and a field programmable gate array (FPGA). Since the types of processor 110 described above are only examples, the type of processor 110 may be configured in various manners within a range understandable to those skilled in the art based on the content of the present disclosure.

The processor 110 may generate a machine learning model for quantifying the quality of electrocardiogram signals based on electrocardiogram data. The processor 110 may generate a machine learning model for quantifying the quality of electrocardiogram signals by using an electrocardiogram dataset labeled with factors that affect the reading of electrocardiogram signals, which is the basis for clinical determinations. In this case, the electrocardiogram dataset used to generate the machine learning model may include at least one of an electrocardiogram dataset labeled based on morphological features of waveforms that affect the reading of electrocardiogram signals and an electrocardiogram dataset labeled based on the analysis of a domain expert on the reading of electrocardiogram signals. The domain expert may be understood as a group or a member of the group that can interpret electrocardiogram signals and perform clinical determinations, such as the diagnosis of a specific disease. That is, the processor 110 may generate a machine learning model that may provide a quantitative index for whether an electrocardiogram signal is a signal having the quality that can be used for clinical reading by utilizing the features identifiable in the waveforms of electrocardiogram signals and the empirical grounds and determinations used to read electrocardiogram signals together.

The processor 110 may estimate the quality of electrocardiogram data to be read by using the machine learning model generated as described above. In this case, the quality of the electrocardiogram data may indicate whether the electrocardiogram data is clinically readable data. Furthermore, the processor 110 may determine whether electrocardiogram data to be read is clinically readable data based on the quality of the electrocardiogram data to be read. More specifically, the processor 110 may generate a quantitative index for the quality of the per-lead signal of the electrocardiogram data to be read by inputting the electrocardiogram data to be read to the machine learning model. Furthermore, the processor 110 may determine whether a signal is missing for each lead of electrocardiogram data to be read by analyzing the waveform of the electrocardiogram data. Furthermore, the processor 110 may determine whether data can be used for clinical reading for each lead of electrocardiogram data to be read by combining the quantitative index, which is generated through the machine learning model, and the results of determining whether a signal for each lead is missing, which are generated through waveform analysis.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize and manage the data required for the processor 110 to perform operations, combinations of data, and the program codes executable by the processor 110. For example, the memory 120 may store electrocardiogram data obtained through the network unit 130 to be described below. The memory 120 may store the program codes that operate the processor 110 to generate a machine learning model, the program codes that operate the processor 110 to estimate the quality of electrocardiogram data by using a generated machine learning model, and various types of data that are generated as program codes are executed.

The network unit 130 according to one embodiment of the present disclosure may be understood as a constituent unit that transmits and receives data via any type of known wired/wireless communication system. For example, the network unit 130 may perform data transmission and reception by using a wired/wireless communication system such as a local area network (LAN), a wideband code division multiple access (WCDMA) network, a long term evolution (LTE) network, the wireless broadband Internet (WiBro), a 5th generation mobile communication (5G) network, an ultra-wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive the data required for the processor 110 to perform computation through wired/wireless communication with any system, any server, any client, or the like. Furthermore, the network unit 130 may transmit the data, generated through the computation of the processor 110, through the wired/wireless communication with any system, any server, any client, or the like. For example, the network unit 130 may receive an electrocardiogram dataset through wired/wireless communication with an electrocardiogram detection device, a database of a medical environment, or the like. The network unit 130 may transmit various types of data, generated through the computation of the processor 110 based on electrocardiogram data, through wired/wireless communication with an electrocardiogram detection device, a database of a medical environment, or the like.

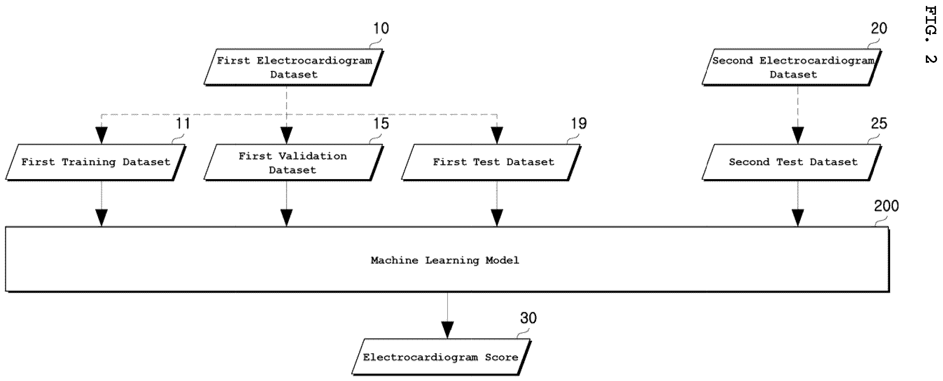

FIG. 2 is a block diagram showing a process of generating a machine learning model according to one embodiment of the present disclosure.

Referring to FIG. 2, the computing device 100 according to one embodiment of the present disclosure may generate a machine learning model 200 for quantifying the quality of electrocardiogram signals based on a first electrocardiogram dataset 10 labeled based on morphological features of electrocardiogram signals and a second electrocardiogram dataset 20 labeled based on the analysis of a domain expert on the reading of electrocardiogram signals. The computing device 100 may generate the machine learning model 200 for estimating the clinical readability of electrocardiogram signals and providing quantitative indices by training machine learning-based candidate models based on the first and second electrocardiogram datasets 10 and 20 and tuning and evaluating hyper parameters of the candidate models. In this case, the first electrocardiogram dataset 10 may be a dataset labeled based on whether electrocardiogram signals are readable, which is analyzed based on the noise identified based on morphological features of electrocardiogram signals. Furthermore, the second electrocardiogram dataset 20 may be a dataset labeled based on the results of analysis by a clinical domain expert on whether electrocardiogram signals can be used for reading a specific disease.

For example, the first electrocardiogram dataset 10 may include labels based on pieces of noise that can be distinguished based on morphological features of the waveforms of electrocardiogram signals. The labels included in the first electrocardiogram dataset 10 may include a first class indicating that an electrocardiogram signal is a readable signal based on the noise identified based on morphological features of the electrocardiogram signal, and a second class indicating that an electrocardiogram signal is an unreadable signal based on the noise identified based on morphological features of the electrocardiogram signal. In this case, the first class and the second class may be distinguished from each other based on whether noise is included to a level at which features used for the clinical reading of an electrocardiogram signal can be identified. More specifically, when the noise that prevents the start and end points of the waveform of an electrocardiogram signal from being identified is present at a predetermined ratio or more, and/or when the noise that prevents the R peak of the electrocardiogram signal from being identified is present at a predetermined ratio or more, the data including the electrocardiogram signal may be labeled as a second class. In this case, the predetermined ratio may be a ratio set by the manufacturer or user of the computing device 100 to achieve the purpose of quantifying the quality. In addition, when the waveform of the electrocardiogram signal has a flat shape, the data including the electrocardiogram signal may be labeled as a second class. When none of the three conditions described above applies, the data including the electrocardiogram signal may be labeled as a first class. In this manner, the electrocardiogram data included in the first electrocardiogram dataset 10 may be labeled based on how the noise identified based on morphological features of an electrocardiogram signal affects the clinical readability of the electrocardiogram signal.

The second electrocardiogram dataset 20 may include labels based on expert analysis on how the noise included in the electrocardiogram signal affects clinical interpretation. The labels included in the second electrocardiogram dataset 20 may include a third class indicating a signal in question is a signal available for reading based on the analysis of a clinical domain expert and a fourth class indicating a signal in question is a signal unavailable for reading based on the analysis of the clinical domain expert. Since the third or fourth class is distinguished through the empirical and intuitive analysis of the domain expert, the second electrocardiogram dataset 20 may be labeled based on the modes of the analyses of the domain expert on whether the noise included in the electrocardiogram signal affects disease reading. In other words, the electrocardiogram data included in the second electrocardiogram dataset 20 may be labeled as the third or fourth class based on the results that appear as modes in big data corresponding to the set of analysis results of a domain expert in order to increase the reliability of labels.

The computing device 100 may use datasets, labeled in different manners, in combination for training, validation, and testing for the generation of the machine learning model 200 to generate a high-quality model. Referring to FIG. 2, the computing device 100 may use one of the first and second electrocardiogram datasets 10 and 20 for the training, validation, and testing of the machine learning model 200 to generate the machine learning model 200. Furthermore, the computing device 100 may use the other one of the first and second electrocardiogram datasets 10 and 20 for the testing of the machine learning model 200. That is, the computing device 100 may use the first and second electrocardiogram datasets 10 and 20 in combination to generate the machine learning model 200, thereby generating a model optimized for quantifying the quality of electrocardiogram signals in such a manner that the performance thereof has been validated according to various criteria.

More specifically, the computing device 100 may divide the first electrocardiogram dataset 10 into a first training dataset 11, a first validation dataset 15, and a first test dataset 19 and separately use them. Furthermore, the computing device 100 may use the second electrocardiogram dataset 20 as a second test dataset 25 to generate the machine learning model 200. That is, the computing device 100 may use a part of the first electrocardiogram dataset 10 for training and validating candidate models for the generation of the machine learning model 200, and may use the second electrocardiogram dataset 20, together with the remaining part of the first electrocardiogram dataset 10, for evaluating at least one candidate model selected through training and the validation. Furthermore, the computing device 100 may generate the machine learning model 200 based on the results of the evaluation for the at least one candidate model performed using the remaining part of the first and second electrocardiogram datasets 10 and 20.

For example, the computing device 100 may divide the first electrocardiogram dataset 10 into the first training dataset 11, the first validation dataset 15, and the first test dataset 19 at the ratio of 8:1:1 and use the divided data to generate the machine learning model 200. The computing device 100 may train candidate models designed with various parameters for the generation of the machine learning model based on the first training dataset 11 included in the first electrocardiogram dataset 10. In this case, the candidate models may be models that receive electrocardiogram data on a per-lead basis and estimate the readability of electrocardiogram signals included in the electrocardiogram data on a per-lead basis. The computing device 100 may perform the performance validation of the trained candidate models based on the first validation dataset 15 included in the first electrocardiogram dataset 10, and may select at least one candidate model through the performance validation. The computing device 100 may evaluate the performance of the at least one candidate model selected through performance validation by using the second electrocardiogram dataset 20, together with the first test dataset 19 included in the first electrocardiogram dataset 10, as the second test dataset 25. Then, the computing device 100 may determine a candidate model, whose evaluation value is higher than a predetermined standard, to be a final candidate model for the generation of the machine learning model 200. Then, the computing device 100 may generate the machine learning model 200 based on the specifications, such as the structure and parameters, of the final candidate model. In this case, the machine learning model 200 may be a model that receives electrocardiogram data on a per-lead basis and outputs an electrocardiogram score 30 indicating the readability of a signal, included in the electrocardiogram data, on a per-lead basis. The electrocardiogram score 30 is an indicator that quantitatively represents a noise evaluation result that reflects the probability value of the readability of the electrocardiogram signal therein, and may be represented by a number, a symbol, or the like. Meanwhile, the ratio of the above-described datasets is only an example, so that the present disclosure is not limited thereto.

In this manner, the computing device 100 may use the overall second electrocardiogram dataset 20, together with a part of the first electrocardiogram dataset 10, for evaluating a model, generated based on a part of the first electrocardiogram dataset 10, in order to reflect the influence of signal noise on clinical reading, such as disease diagnosis, in the quantification of the quality of the signal. In other words, the computing device 100 may use the overall second electrocardiogram dataset 20 to evaluate a model generated based on the first electrocardiogram dataset 10, thereby allowing the quality of the signal estimated by the machine learning model 200 to reflect whether the electrocardiogram signal is clinically readable therein. Through this generation of the machine learning model 200, the computing device 100 may quantify the quality of the signal so that the quality of the signal can indicate the clinical readability of the signal, rather than simply evaluating the quality of the signal with noise or artifacts of the signal.

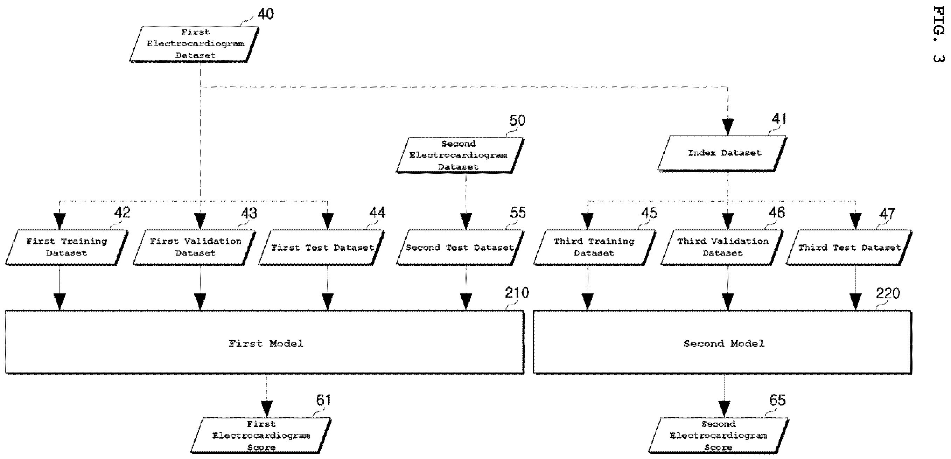

FIG. 3 is a block diagram showing a process of generating a machine learning model according to an alternative embodiment of the present disclosure.

Referring to FIG. 3, a machine learning model according to an alternative embodiment of the present disclosure may include a first model 210 based on a neural network for estimating the readability of an electrocardiogram signal based on an electrocardiogram dataset, and a second model 220 based on regression analysis for estimating the readability of an electrocardiogram signal based on an electrocardiogram dataset. More specifically, according to the alternative embodiment of the present disclosure, the computing device 100 may generate a machine learning model by ensembling the first model 210 based on a neural network and the second model 220 based on regression analysis.

For example, the first model 210 may include a convolutional neural network based on ResNet. This first model 210 may be generated through the following process. First, candidate models of the first model 210 may receive a first training dataset 42 and be trained with it. In this case, training may be performed based on supervised learning using labels based on morphological features of electrocardiogram signals included in the first training dataset 42. When the training of the candidate models of the first model 210 is completed, the candidate models of the first model 210 may receive a first validation dataset 43 and be validated for their performance. The candidate models of the first model 210 selected through the performance validation may receive a first test dataset 44 and a second test dataset 55 be evaluated for their performance. Then, based on the specifications of the candidate model that has desirable performance for both the first test dataset 44 and the second test dataset 55 through the performance evaluation, the first model 210 may be generated. In this case, the first model 210 may be a model that receives electrocardiogram data and outputs a first electrocardiogram score 61 indicating the readability of the per-lead signal included in the electrocardiogram data. The first electrocardiogram score 61 may be a concept corresponding to the electrocardiogram score 30 of FIG. 2 described above.

The second model 220 may include a model based on logistic regression analysis. This second model 220 may be generated through the following process. First, candidate models of the second model 220 may use an index dataset 41, extracted from the first electrocardiogram dataset 40, as input. The index dataset 41 may be a dataset generated by extracting information about signal quality indices (zerocrossSQI, minSQI, maxSQI, powerSQI, q1SQI, q3SQI, sSQI, kSQI, highfreqSQI, baseSQI, pSQI, etc.) that may be used to evaluate noise in electrocardiogram signals. That is, the candidate models of the second model 220 may receive the third training dataset 45 of the index dataset 41 that includes mathematically clearly expressible signal features according to the signal quality indices for noise evaluation and be trained with it. When the training of the candidate models of the second model 220 is completed, the candidate models of the second model 220 may receive a third validation dataset 46 and be validated for their performance. The candidate models of the second model 220 selected through the performance validation may receive a third test dataset 47 and be evaluated for their performance. Furthermore, based on the specifications of the candidate model that has desirable performance for the third test dataset 47 through the performance evaluation, the second model 220 may be generated. In this case, the second model 220 may be a model that receives index data extracted from the electrocardiogram data and outputs a second electrocardiogram score 65 indicating the readability of the per-lead signal included in the electrocardiogram signal. The second electrocardiogram score 65 may be a concept corresponding to the electrocardiogram score 30 of FIG. 2 described above.

In this manner, the computing device 100 may expect generalized performance for external data, other than the datasets on which learning and evaluation were performed, by using the second model 220 based on regression analysis together with the first model 210 based on a neural network in order to minimize the overfitting problem, which is a limitation of the first model 210 based on a neural network. Furthermore, since the second model 220 receives the features extracted by clear mathematical expressions and the determination processes and bases for making decisions based on criteria may be identified, the computing device 100 may overcome the limitation of the first model 210, in which it is difficult to identify the bases for determinations, by using the second model 220 together with the first model.

In addition, the first model 210 based on a neural network may be viewed as unreliable because the bases for determinations are unknown. The computing device 100 may provide minimum safety measures in terms of reliability by generating a machine learning model by ensembling the first model 210 and the second model 220. Furthermore, in general, the first model 210 based on a neural network is considered to have high accuracy but low basis, and the second model 220 based on regression analysis is considered to have low accuracy but clear basis. Accordingly, the computing device 100 may customize and generate a final result value according to a user's desired purpose based on the ensemble form of the first model 210 and the second model 220.

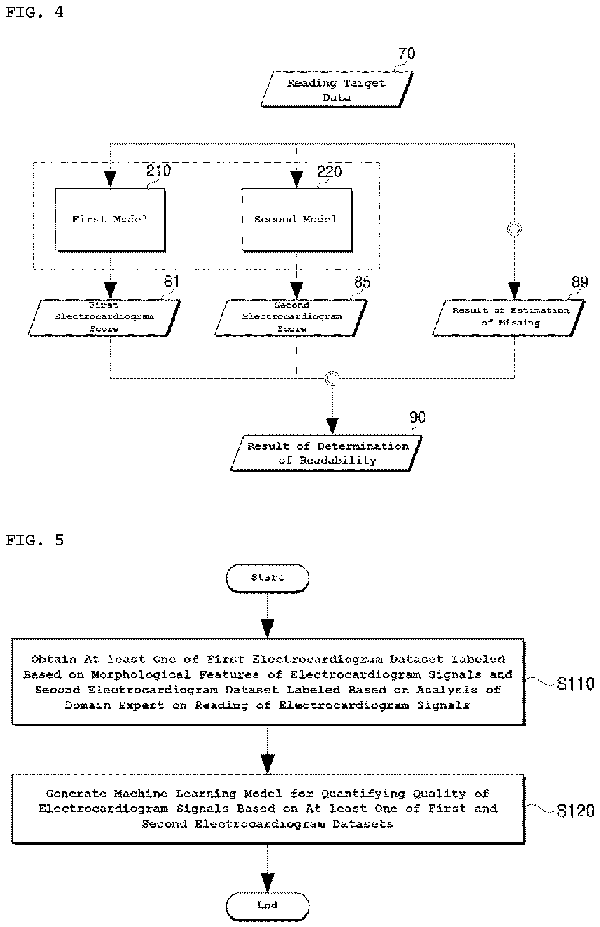

FIG. 4 is a block diagram showing a process in which a computing device quantifies the quality of biosignals according to one embodiment of the present disclosure.

Referring to FIG. 4, the computing device 100 according to one embodiment of the present disclosure may compute a first electrocardiogram score 81 indicating readability for each lead of reading target data 70 by inputting the reading target data 70 to the first model 210 included in the machine learning model. In this case, the first model 210 may be generated based on at least one of an electrocardiogram dataset labeled based on morphological features of the electrocardiogram signal and an electrocardiogram dataset labeled based on the analysis of a domain expert on the reading of the electrocardiogram signal. Furthermore, although not illustrated in FIG. 4, the computing device 100 may extract index data, including information about signal quality indices regarding the noise of electrocardiogram signals, from the reading target data 70. Furthermore, the computing device 100 may compute a second electrocardiogram score 85 indicating readability for each lead of the reading target data 70 by inputting the index data, extracted from the reading target data 70, to the second model 220 included in the machine learning model. The second model 220 may be generated based on the index dataset extracted from the electrocardiogram dataset labeled based on the morphological features of the electrocardiogram signals.

Furthermore, the computing device 100 may estimate whether the signal included in the reading target data 70 is missing. The computing device 100 may estimate whether the signal is missing for all leads included in the reading target data 70 and generate a result 89 of estimating whether the signal is missing for each lead. More specifically, whether the signal is missing may be estimated based on whether the per-lead signal value of the reading target data 70 is blank at a predetermined ratio or more or whether the waveform of the per-lead signal of the reading target data 70 is flat. In this case, the predetermined ratio may be a ratio set by the manufacturer or user of the computing device 100 to achieve the purpose of quantifying the quality.

The computing device 100 may determine whether the reading target data 70 is readable data by combining the first electrocardiogram score 81, the second electrocardiogram score 85, and the result 89 of estimating whether the signal is missing. The computing device 100 may generate the result 90 of determining whether the reading target data 70 is readable for each lead of the reading target data 70 by comprehensively analyzing the output value of the machine learning model and the result of estimation of whether the signal is missing for each lead of the reading target data 70. For example, the computing device 100 may determine that noise is present in the signal of the lead higher than a first threshold value by comparing the first electrocardiogram score 81 with the first threshold value. Furthermore, the computing device 100 may determine that noise is present in the signal of the lead higher than a second threshold value by comparing the second electrocardiogram score 85 with the second threshold value. In this case, the first threshold value and the second threshold value may be values predetermined by the manufacturer or user of the computing device 100 according to the purpose, and may be the same or different values. Furthermore, the computing device 100 may determine that the signal of the lead, determined to be missing according to the result 89 of estimating whether the signal is missing for each lead, has noise, and may determine that the signal of the lead determined to be not missing has no noise. When it is determined that noise is present in the signal of the lead in at least one of a determination result based on a first electrocardiogram score 81, a determination result based on the second electrocardiogram score 85 and a determination result based on the result 89 of estimating whether the signal is missing for each lead, the computing device 100 may determine that the signal of the corresponding lead is unreadable data. In this manner, the computing device 100 may provide quantitative information about the quality of the signal having high reliability by complementarily using the analysis results that can be utilized to determine the readability.

FIG. 5 is a flowchart showing a method of generating a machine learning model for quantifying the quality of biosignals according to one embodiment of the present disclosure.

Referring to FIG. 5, the computing device 100 according to one embodiment of the present disclosure may obtain at least one of a first electrocardiogram dataset labeled based on morphological features of electrocardiogram signals and a second electrocardiogram dataset labeled based on the analysis of a domain expert on the reading of electrocardiogram signals in step S110. In this case, the first electrocardiogram data set may be labeled as a first class indicating that the electrocardiogram signal is a readable signal, or a second class indicating that the electrocardiogram signal is an unreadable signal, according to the noise identified based on morphological features of the electrocardiogram signal. Furthermore, the second class may correspond to at least one of a case where noise that prevents at least one of the start and end points of the electrocardiogram signal from being identified is present at a predetermined ratio or more and a case where noise that prevents the R peak of the electrocardiogram signal from being identified is present at a predetermined ratio or more. The second electrocardiogram dataset may be labeled based on the modes of the analyses of a domain expert on whether the noise present in the electrocardiogram signal affects disease diagnosis. For example, the computing device 100 may obtain at least one of the first electrocardiogram dataset and the second electrocardiogram dataset through wired/wireless communication with a client for labeling electrocardiogram data. The computing device 100 may be equipped with an input/output unit, and may directly perform the labeling of the electrocardiogram data and generate at least one of the first electrocardiogram dataset and the second electrocardiogram dataset.

The computing device 100 may generate a machine learning model for quantifying the quality of electrocardiogram signals based on at least one of the first and second electrocardiogram datasets, obtained through step S110, in step S120. The computing device 100 may divide the first electrocardiogram dataset into a training dataset, a validation dataset, and a test dataset and separately use them in order to generate the machine learning model. Furthermore, the computing device 100 may use the second electrocardiogram dataset as a test dataset in order to generate the machine learning model. In this case, the machine learning model is a model that outputs the readability of the electrocardiogram signal as a quantified index based on per-lead electrocardiogram dataset, and may include a first model based on a neural network and a second model based on regression analysis.

For example, the computing device 100 may train candidate models of the first model based on a first training dataset included in the first electrocardiogram dataset. The computing device 100 may validate the performance of the trained candidate models based on a first validation dataset included in the first electrocardiogram dataset. The computing device 100 may evaluate the performance of at least one candidate model, selected through the validation, based on a first test dataset which is included in the first electrocardiogram dataset and a second test dataset which is the second electrocardiogram dataset. The computing device 100 may identify a model having desirable performance for the data labeled based on different features and criteria by utilizing both the first test dataset and the second test dataset for evaluation. In this case, the model having desirable performance identified by the computing device 100 may be a model having the highest performance evaluation index or a model having a value equal to or higher than a specific reference value. The computing device 100 may generate a first model based on the specifications of the candidate model identified through the evaluation. In this case, the specifications of the model are information about parameters for the construction of a neural network, and can include the size, depth, width, training rate, etc. of the kernel.

The computing device 100 may generate an index dataset by extracting information about signal quality indices used to evaluate the noise of electrocardiogram signals from the first electrocardiogram dataset. That is, the computing device 100 may generate the index dataset based on features representing signal quality indices according to the noise of electrocardiogram signals from the first electrocardiogram dataset. The computing device 100 may train candidate models of the second model based on a third training dataset included in the generated index dataset. The computing device 100 may validate the performance of the trained candidate models based on a third validation dataset included in the generated index dataset. The computing device 100 may evaluate the performance of at least one candidate model, selected through the validation, based on a third test dataset included in the generated index dataset. The computing device 100 may identify a model having desirable performance based on the third test dataset. In this case, the model having desirable performance identified by the computing device 100 may be a model having the highest performance evaluation index or a model having a value equal to or higher than a specific reference value. The computing device 100 may generate a second model based on the specifications of the candidate model identified through the evaluation. In this case, the specifications of the model may be information about parameters of a model for performing logistic regression analysis.

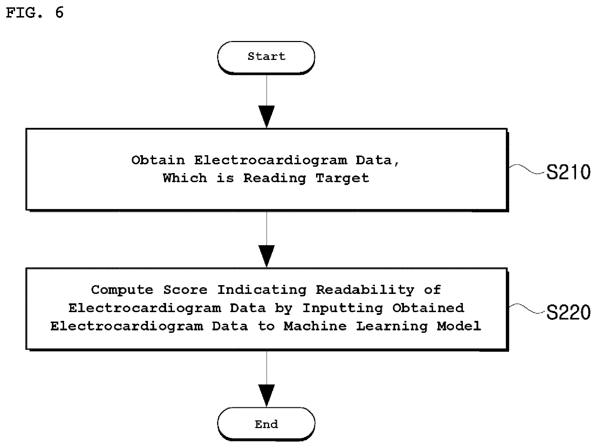

FIG. 6 is a flowchart showing a method of quantifying the quality of biosignals according to one embodiment of the present disclosure.

Referring to FIG. 6, the computing device 100 according to one embodiment of the present disclosure may obtain reading target data in step S210. The reading target data may be understood as electrocardiogram data generated to be used for clinical reading, such as diagnosis or prediction of a specific disease. For example, the computing device 100 may receive the reading target data, generated in an electrocardiogram detection device, through wired/wireless communication with the electrocardiogram detection device.

The computing device 100 may compute a score indicating the readability of the reading target data by inputting the reading target data, obtained through step S210, to a machine learning model in step S220. In this case, the machine learning model may be a model generated based on an electrocardiogram dataset labeled based on morphological features of electrocardiogram signals according to FIG. 5 described above and an electrocardiogram dataset labeled based on the analysis of a domain expert on the reading of electrocardiogram signals. For example, the computing device 100 may compute a first score indicating noise reflecting readability therein by inputting the reading target data to a first model based on a neural network included in the machine learning model. Furthermore, the computing device 100 may compute a second score indicating noise reflecting readability therein by inputting the reading target data to a second model based on regression analysis included in the machine learning model.

Meanwhile, the computing device 100 may estimate whether the signal included in the reading target data obtained through step S210 is missing. The estimation of whether the signal is missing may be performed in parallel with step S220 of computing the score through machine learning. For example, when the signal value is blank at 50% or higher based on a specific lead of the reading target data, the computing device 100 may estimate that the signal of the corresponding lead is missing. Furthermore, when the waveform of the signal is flat based on a specific lead of the reading target data, the computing device 100 may estimate that the signal of the corresponding lead is missing. Since the above-described number '50' is only an example, the numerical value of the ratio for the determination of the blank may be changed in accordance with the purpose of use of the computing device 100.

The computing device 100 may determine whether the reading target data is readable data by combining the score calculated through step S220 and the presence or absence of the missing signal estimated through the above-described process. The computing device 100 may determine whether noise is present in the signal included in the reading target data by comparing the score calculated through step S220 with a threshold value. Furthermore, the computing device 100 may determine whether the reading target data is readable data based on at least one of the presence or absence of noise in the signal, determined based on the score, and the presence or absence of the missing signal. For example, the computing device 100 may determine whether noise is present in the signal for each lead of the reading target data by comparing the first score, generated through the first model based on a neural network, with a threshold value. The computing device 100 may determine whether noise is present in the signal for each lead of the reading target data by comparing the second score, generated through the second model based on regression analysis, with a threshold value. Furthermore, the computing device 100 may determine whether noise is present in the signal for each lead based on whether the signal for each lead is missing. The computing device 100 may synthesize the results of individual determinations for whether noise is present, and may determine whether clinical reading is possible for each lead of the reading target data.

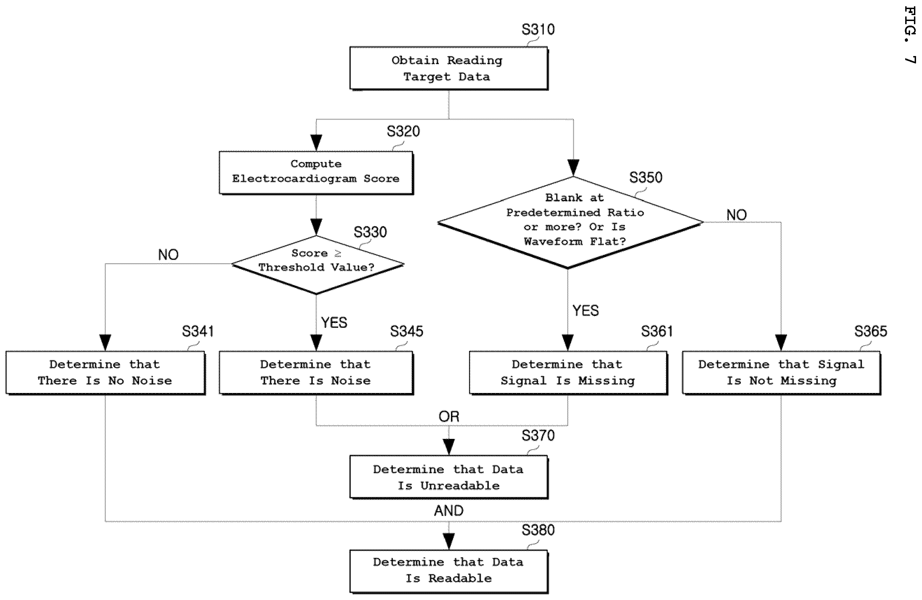

FIG. 7 is a flowchart summarizing a process of quantifying the quality of biosignals according to one embodiment of the present disclosure. Since steps S310 and S320 of FIG. 7 correspond to respective corresponding steps of FIG. 6 described above, descriptions thereof will be omitted below.

Referring to FIG. 7, the computing device 100 according to one embodiment of the present disclosure may determine whether the score computed by a machine learning model for reading target data is equal to or higher than a threshold value in step S330. When the score is lower than the threshold value based on a specific lead, the computing device 100 may determine that noise is not present in the signal of the corresponding lead in step S341. Conversely, when the score is equal to or higher than the threshold value based on a specific lead, the computing device 100 may determine that noise is present in the signal of the corresponding lead in step S345. When the machine learning model includes a first model based on a neural network and a second model based on regression analysis, the computing device 100 may determine whether noise is present by individually compare the score of each of the models with a threshold value. In this case, the threshold value compared with the score calculated by the first model and the threshold value compared with the score calculated by the second model may be the same or different.

The computing device 100 may determine whether a signal is missing based on whether the signal of each lead of the reading target data is blank at a predetermined ratio or more or whether the waveform of the signal of each lead is flat in step S350. When the signal of the specific lead of the reading target data is blank at the predetermined ratio or more or the waveform of the signal of the specific lead is flat, the computing device 100 may determine that the signal of the corresponding lead is missing in step S361. Furthermore, the computing device 100 may determine that noise is not present in the signal of the lead whose signal is missing. Conversely, when the signal of the specific lead of the reading target data is blank at a ratio less than the predetermined ratio or the waveform of the signal of the specific lead is not flat, the computing device 100 may determine that the signal of the corresponding lead is not missing in step S365. Furthermore, the computing device 100 may determine that noise is not present in the signal of the lead whose signal is not missing.

When it is determined that noise is present in a specific lead through the above-described process, the computing device 100 may determine that the signal of the specific lead is not clinically readable in step S370. That is, when it is determined that noise is present in step S345 because the score is estimated to be equal to or higher than a threshold value based on the specific lead or when it is determined that the signal is missing in step S361, the computing device 100 may determine that the signal of the corresponding lead is not readable in step S370. Conversely, when it is determined that noise is not present in the specific lead through the above-described process, the computing device 100 may determine that the signal of the specific lead is clinically readable in step S380. That is, when it is determined that noise is not present in step S341 because the score is estimated to be lower than the threshold value based on the specific lead or when it is determined that a signal is not missing in step S365, the computing device 100 may determine that the signal of the corresponding lead is readable in step S380.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of quantifying quality of biosignals, the method being performed by a computing device including at least one processor, the method comprising:
obtaining at least one of a first electrocardiogram dataset labeled based on morphological features of electrocardiogram signals and a second electrocardiogram dataset labeled based on analysis of a domain expert on reading of electrocardiogram signals; and
generating a machine learning model for quantifying quality of electrocardiogram signals based on at least one of the first and second electrocardiogram datasets.

2. The method of claim 1, wherein the first electrocardiogram dataset is labeled as a first class indicating that an electrocardiogram signal is a readable signal or a second class indicating that an electrocardiogram signal is an unreadable signal, based on noise identified based on morphological features of the electrocardiogram signal.

3. The method of claim 2, wherein the second class corresponds to at least one of:
a case where noise that prevents at least one of start and end points of a waveform of the electrocardiogram signal from being identified is present at a predetermined ratio or more; and
a case where noise that prevents an R peak of the electrocardiogram signal from being identified is present at a predetermined ratio or more.

4. The method of claim 1, wherein the second electrocardiogram dataset is labeled based on modes of analyses of the domain expert on whether noise present in the electrocardiogram signals affects diagnosis of disease.

5. The method of claim 1, wherein:
any one of the first and second electrocardiogram datasets is divided into and used as a training dataset, a validation dataset, and a test dataset to generate the machine learning model; and
a remaining one of the first and second electrocardiogram datasets is used as a test dataset to generate the machine learning model.

6. The method of claim 1, wherein the machine learning model comprises:
a first model based on a neural network for estimating readability of electrocardiogram signals based on an electrocardiogram dataset; and
a second model based on regression analysis for estimating readability of electrocardiogram signals based on an electrocardiogram dataset.

7. The method of claim 6, wherein generating the machine learning model for quantifying the quality of electrocardiogram signals based on at least one of the first and second electrocardiogram datasets comprises:
training candidate models of the first model based on a first training dataset included in the first electrocardiogram dataset;
validating performance of the trained candidate models based on a first validation dataset included in the first electrocardiogram dataset;
evaluating performance of at least one candidate model, selected through the validation, based on a first test dataset which is included in the first electrocardiogram dataset and a second test dataset which is the second electrocardiogram dataset; and
generating the first model based on specifications of the candidate model identified through the evaluation.

8. The method of claim 6, wherein generating the machine learning model for quantifying the quality of electrocardiogram signals based on at least one of the first and second electrocardiogram datasets comprises:
extracting information about signal quality indices (SQI) used to evaluate noise of electrocardiogram signals from the first electrocardiogram dataset, and generating an index dataset;
training candidate models of the second model based on a third training dataset included in the generated index dataset;
validating performance of the trained candidate models based on a third validation dataset included in the generated index dataset;
evaluating performance of at least one candidate model, selected through the validation, based on a third test dataset included in the generated index dataset; and
generating the second model based on specifications of the candidate model identified through the evaluation.

9. A method of quantifying quality of biosignals, the method being performed by a computing device including at least one processor, the method comprising:
obtaining reading target data; and
computing a score indicating readability of the reading target data by inputting the obtained reading target data to a machine learning model;
wherein the machine learning model is generated based on at least one of a first electrocardiogram dataset labeled based on morphological features of electrocardiogram signals and a second electrocardiogram dataset labeled based on analysis of a domain expert on reading of electrocardiogram signals.

10. The method of claim 9, further comprising:
estimating whether a signal included in the obtained reading target data is missing; and
determining whether the obtained reading target data is readable data by combining the computed score and the estimated presence or absence of a missing signal.

11. The method of claim 10, wherein the presence or absence of a missing signal is estimated based on whether a signal value included in the obtained reading target data is blank at a predetermined ratio or more or whether a waveform of a signal included in the obtained reading target data is flat.

12. The method of claim 10, wherein determining whether the obtained reading target data is readable data by combining the computed score and the estimated presence or absence of a missing signal comprises:
determining whether noise is present in a signal included in the obtained reading target data by comparing the computed score with a threshold value; and
determining whether the obtained reading target data is readable data based on at least one of presence or absence of noise in the signal determined based on the computed score and the presence or absence of a missing signal estimated above.

13. The method of claim 12, wherein determining whether noise is present in the signal included in the obtained reading target data by comparing the computed score with the threshold value comprises:
determining that noise is present in a signal of a specific lead when a computed score based on the specific lead is equal to or higher than the threshold value.

14. The method of claim 12, wherein determining whether the obtained reading target data is readable data based on at least one of the presence or absence of noise in the signal determined based on the computed score and the presence or absence of a missing signal estimated above comprises:
determining that a signal of a specific lead determined to have noise based on the computed score or estimated as a mission signal is unreadable data.

15. A computer program stored in a computer-readable storage medium, the computer program causing operations for quantifying quality of biosignals to be performed when executed by at least one processor, wherein the operations comprise operations of:
obtaining at least one of a first electrocardiogram dataset labeled based on morphological features of electrocardiogram signals and a second electrocardiogram dataset labeled based on analysis of a domain expert on reading of electrocardiogram signals; and
generating a machine learning model for quantifying quality of electrocardiogram signals based on at least one of the first and second electrocardiogram datasets.

16. A computing device for quantifying quality of biosignals, the computing device comprising:
a processor including at least one core;
memory including program codes executable by the processor; and
a network unit for obtaining at least one of a first electrocardiogram dataset labeled based on morphological features of electrocardiogram signals and a second electrocardiogram dataset labeled based on analysis of a domain expert on reading of electrocardiogram signals; and
wherein the processor:
generates a machine learning model for quantifying quality of electrocardiogram signals based on at least one of the first and second electrocardiogram datasets.
